# EUROPEAN PATENT APPLICATION

(11) **EP 4 400 116 A1**
(43) Date of publication of application: **17.07.2024**
(21) Application number: 22870316.1
(22) Date of filing: 15.09.2022
(51) Int. Cl.: A61K 39/395, A61K 9/08, A61K 47/02, A61K 47/12, A61K 47/26, C07K 16/24, A61K 39/00

(54) **PHARMACEUTICAL COMPOSITION CONTAINING ANTI-TNF ALPHA ANTIBODY**

(30) Priority: 16.09.2021 KR 20210124326
(71) Applicant: APROGEN Inc., Seongnam-si, Gyeonggi-do 13215 (KR)
(72) Inventor: PARK, Mijeong, Seoul 06276 (KR); JIN, Seok Min, Gyeonggi-do 16804 (KR)
(74) Representative: Manitz Finsterwald Patent- und Rechtsanwaltspartnerschaft mbB
(86) International application number: PCT/KR2022/013800
(87) International publication number: WO 2023/043232

(57) **Abstract**

Provided is a stable pharmaceutical composition containing an anti-TNFα antibody. The pharmaceutical composition is characterized by being stable while containing a high concentration of the anti-TNFα antibody.

## Description

### Technical Field

### CROSS-REFERENCE TO RELATED APPLICATION(S)

This patent application claims priority to and the benefit of Korean Patent Application No. 10-2021-0124326 filed with the Korean Intellectual Property Office on September 16, 2021, the disclosure of which is incorporated herein by reference in its entirety

This application pertains to a stable pharmaceutical composition containing an anti-TNFα antibody at a high concentration.

### Background Art

Tumor necrosis factor alpha (TNFα) is a naturally occurring mammalian cytokine produced by various cell types including monocytes and macrophages in response to endotoxins or other stimuli. TNFα is a major mediator of inflammation, immunity, and pathophysiological responses.

Elevated levels of TNFα are associated with various diseases. For example, TNFα is upregulated in various chronic disease such as rheumatoid arthritis (RA), inflammatory bowel diseases including Crohn's disease and ulcerative colitis. It is also upregulated in conditions such as sepsis, congestive heart failure, asthma, and multiple sclerosis, etc.

Adalimumab (Humira^{®}, AbbVie, Inc.) is a recombinant human IgG1 monoclonal antibody specific for human TNFα. This antibody is also known as D2E7. Adalimumab is disclosed and claimed in U.S. Patent No. 6,090,382. Adalimumab specifically binds to TNFα and neutralizes the biological function thereof by blocking the interaction with p55 and p75 cell surface TNFα receptors.

The development of a stable liquid formulation of Adalimumab that can be stored for extended periods without significant loss of efficacy is needed.

### Disclosure

### Technical Problem

The present application provides an aqueous liquid composition for enhancing the stability of a protein drug, particularly an antibody, more particularly an anti-TNFα antibody.

Specifically, an embodiment provides a pharmaceutical composition containing:
80 mg/ml to 200 mg/ml of an anti-TNFα antibody;
a buffer;
an excipient; and
a surfactant,
wherein the composition has a pH of 4 to 7.5.

Another embodiment provides a method for producing a liquid formulation of a stable anti-TNFα antibody, the method including a step of mixing
80 mg/ml to 200 mg/ml of an anti-TNFα antibody;
a buffer;
an excipient; and
a surfactant to prepare an aqueous liquid composition,
wherein the aqueous liquid composition has a pH of 4 to 7.5.

Another embodiment provides a method for enhancing the stability of an anti-TNFα antibody in a liquid formulation, the method including a step of mixing:
80 mg/ml to 200 mg/ml of the anti-TNFα antibody;
a buffer;
an excipient; and
a surfactant to prepare an aqueous liquid composition,
wherein the aqueous liquid composition has a pH of 4 to 7.5.

In the composition and methods, the buffer may be at least one selected from the group consisting of phosphate, succinate, acetate, and pharmaceutically acceptable salts thereof.

In the composition and methods, the excipient may be at least one selected from the group consisting of mannitol, glycerol, glycine, and pharmaceutically acceptable salts thereof.

In the composition and methods, the surfactant may be polysorbate 20.

In the composition and methods, the anti-TNFα antibody may be adalimumab. The adalimumab may be contained at a concentration of 80 mg/ml, 100 mg/ml, 120 mg/ml, 150 mg/ml, or 200 mg/ml, for example, 100 mg/ml.

### Technical Solution

This application provides an aqueous liquid composition with enhanced stability of an antibody (aqueous liquid antibody formulation). The aqueous liquid composition may include the components listed below and the balance of an aqueous medium (water (purified water), saline, injectable water, etc.).

As used herein, the terms "aqueous liquid composition", "pharmaceutical composition", "formulation", "liquid formulation", and "liquid antibody formulation" all refer to a composition containing an antibody (e.g., adalimumab) and can be used interchangeably.

Below, a detailed description will be given of the present application.

### Anti-TNFα antibody

Herein, the anti-TNFα antibody means an antibody that binds to TNFα to modulate the biological activity thereof.

The anti-TNFα antibody may be in the form of a full-length antibody or an antibody fragment possessing the antigen-binding site, but is not particularly limited thereto. Specifically, the anti-TNFα antibody may be a human immunoglobulin G1 monoclonal antibody and, more specifically, may be adalimumab (human IgG1 kappa).

Adalimumab, also known as D2E7, is sold under the trade name Humira. Humira has been approved for sale as a treatment for rheumatoid arthritis and is used for the treatment of Crohn's disease, ankylosing spondylitis, psoriatic arthritis, ulcerative colitis, and other conditions. Information on adalimumab can be easily obtained by those skilled in the art from publicly available databases.

The pharmaceutical composition provided herein may contain adalimumab in a therapeutically effective amount. Specifically, the concentration (content) of adalimumab in the pharmaceutical composition may be in the range of about 80 mg/ml to about 200 mg/ml, about 80 mg/ml to about 150 mg/ml, about 80 mg/ml to about 120 mg/ml, about 80 mg/ml to about 100 mg/ml, about 100 mg/ml to about 200 mg/ml, about 100 mg/ml to about 150 mg/ml, or about 100 mg/ml to about 120 mg/ml, for example, about 80 mg/ml, about 100 mg/ml, about 120 mg/ml, about 150 mg/ml, or about 200 mg/ml.

The pharmaceutical composition provided herein exhibits excellent stability even when containing antibodies at relatively high concentrations (specifically, 80 mg/ml or more, 100 mg/ml or more, 120 mg/ml or more, or 150 mg/ml or more).

### Buffer

In antibody formulations, the buffer can function to adjust the pH of the formulation. The buffer that can be contained in the pharmaceutical composition provided herein may-include one or more selected from the group consisting of succinate, phosphate, acetate, and pharmaceutically acceptable salts thereof, and can be used in an aqueous state. The pharmaceutically acceptable salts may include one or more selected from the group consisting of sodium salts (e.g., sodium phosphate, sodium succinate, etc.) and potassium salts, but are not limited thereto.

In an embodiment, the buffer may include one or more selected from the group consisting of succinate, phosphate, and pharmaceutically acceptable salts thereof, and not include one or more selected from the group consisting of acetate, histidine, citrate, and pharmaceutically acceptable salts thereof (e.g., all of these). In another example, the buffer may include one or more selected from the group consisting of succinate and its pharmaceutically acceptable salts, and exclude one or more (e.g., all) selected from the group consisting of phosphate, histidine, acetate, citrate, and pharmaceutically acceptable salts thereof.

The buffer may be contained at a concentration of about 1 mM to about 50 mM, about 1 mM to about 40 mM, about 1 mM to about 30 mM, about 1 mM to about 20 mM, about 1 mM to about 10 mM, about 3 mM to about 50 mM, about 3 mM to about 40 mM, about 3 mM to about 30 mM, about 3 mM to about 20 mM, about 3 mM to about 10 mM, about 5 mM to about 50 mM, about 5 mM to about 40 mM, about 5 mM to about 30 mM, about 5 mM to about 20 mM, about 5 mM to about 10 mM, about 8 mM to about 50 mM, about 8 mM to about 40 mM, about 8 mM to about 30 mM, about 8 mM to about 20 mM, about 8 mM to about 10 mM, about 10 mM to about 50 mM, about 10 mM to about 40 mM, about 10 mM to about 30 mM, or about 10 mM to about 20 mM, for example, about 10 mM, or about 20mM, based on the total amount of the pharmaceutical composition.

### Excipient

The pharmaceutical composition provided herein may include one or more excipients selected from the group consisting of mannitol, glycerol, glycine, glucose, and glutamic acid. More specifically, the excipient may include mannitol solely; mannitol and at least one selected from the group consisting of glycerol, glycine, glucose, and glutamic acid; or mannitol and at least one selected from the group consisting of glycerol and glycine. In one specific embodiment, the excipient may include mannitol, a combination of mannitol and glycerol, or a combination of mannitol and glycine. In this regard, the excipient may include mannitol, a combination of mannitol and glycerol, or a combination of mannitol and glycine, and may exclude glucose. In another embodiment, the excipient may include mannitol, a combination of mannitol and glycerol, or a combination of mannitol and glycine, with the exclusion of glucose and glutamic acid.

The excipient may be contained at a concentration of about 5mM to about 350mM, about 5mM to about 300mM, about 5mM to about 270mM, about 10mm to about 350mM, about 10mM to about 300mM, about 10mM to about 270mM, about 20mM to about 350mM, about 20mM to about 300mM, about 20mM to about 270mM, about 25mM to about 350mM, about 25mM to about 300mM, about 25mM to about 270mM, about 50mM to about 350mM, about 50mM to about 300mM, about 50mM to about 270mM, about 75mM to about 350mM, about 75mM to about 300mM, about 75mM to about 270mM, about 100mM to about 350mM, about 100mM to about 300mM, or about 100mM to about 270mM, based on the total amount of the pharmaceutical composition.

For example, mannitol may be contained at a concentration of about 5.5mM to about 55mM, about 5.5mM to about 41.25mM, about 5.5mM to about 27.5mM, about 5.5mM to about 22mM, about 5.5mM to about 16.5mM, about 5.5mM to about 14mM, about 8.25mM to about 55mM, about 8.25mM to about 41.25mM, about 8.25mM to about 27.5mM, about 8.25mM to about 22mM, about 8.25mM to about 16.5mM, about 8.25mM to about 14mM, or about 11mM (or 0.1%(w/v) to 1%(w/v), 0.1%(w/v) to 0.75%(w/v), 0.1%(w/v) to 0.5%(w/v), 0.1%(w/v) to 0.4%(w/v), 0.1%(w/v) to 0.3%(w/v), 0.1%(w/v) to 0.25%(w/v), 0.15%(w/v) to 1%(w/v), 0.15%(w/v) to 0.75%(w/v), 0.15%(w/v) to 0.5%(w/v), 0.15%(w/v) to 0.4%(w/v), 0.15%(w/v) to 0.3%(w/v), 0.15%(w/v) to 0.25%(w/v), or 0.2%(w/v)), based on the total amount of the pharmaceutical composition.

Glycine may be contained at a concentration of about 133mM to about 400mM, about 133mM to about 333mM, about 133mM to about 293mM, about 133mM to about 266mM, about 239mM to about 400mM, about 239mM to about 333mM, about 239mM to about 293mM, about 239mM to about 266mM, about 266mM to about 400mM, about 266mM to about 333mM, about 266mM to about 293mM, or about 266mM (or 1%(w/v) to 3%(w/v), 1%(w/v) to 2.5%(w/v), 1%(w/v) to 2.2%(w/v), 1%(w/v) to 2%(w/v), 1.8%(w/v) to 3%(w/v), 1.8%(w/v) to 2.5%(w/v), 1.8%(w/v) to 2.2%(w/v), 1.8%(w/v) to 2%(w/v), 2%(w/v) to 3%(w/v), 2%(w/v) to 2.5%(w/v), 2%(w/v) to 2.2%(w/v), or 2%(w/v)), based on the total amount of the pharmaceutical composition.

Glycerol may be contained at a concentration of about 162mM to about 326mM, about 162mM to about 300mM, about 162mM to about 272mM, about 162mM to about 239mM, about 217mM to about 326mM, about 217mM to about 300mM, about 217mM to about 272mM, about 217mM to about 239mM, about 239mM to about 326mM, about 239mM to about 300mM, about 239mM to about 272mM, or about 239mM (or 1.5%(w/v) to 3%(w/v), 1.5%(w/v) to 2.75%(w/v), 1.5%(w/v) to 2.5%(w/v), 1.5%(w/v) to 2.2%(w/v), 2%(w/v) to 3%(w/v), 2%(w/v) to 2.75%(w/v), 2%(w/v) to 2.5%(w/v), 2%(w/v) to 2.2%(w/v), 2.2%(w/v) to 3%(w/v), 2.2%(w/v) to 2.75%(w/v), 2.2%(w/v) to 2.5%(w/v), or 2.2%(w/v)), based on the total amount of the pharmaceutical composition.

In an embodiment, the pharmaceutical composition may include, as an excipient,
(1) mannitol at a concentration of about 5.5mM to about 55mM, about 5.5mM to about 41.25mM, about 5.5mM to about 27.5mM, about 5.5mM to about 22mM, about 5.5mM to about 16.5mM, about 5.5mM to about 14mM, about 8.25mM to about 55mM, about 8.25mM to about 41.25mM, about 8.25mM to about 27.5mM, about 8.25mM to about 22mM, about 8.25mM to about 16.5mM, about 8.25mM to about 14mM, or about 11mM (or 0.1%(w/v) to 1%(w/v), 0.1%(w/v) to 0.75%(w/v), 0.1%(w/v) to 0.5%(w/v), 0.1%(w/v) to 0.4%(w/v), 0.1%(w/v) to 0.3%(w/v), 0.1%(w/v) to 0.25%(w/v), 0.15%(w/v) to 1%(w/v), 0.15%(w/v) to 0.75%(w/v), 0.15%(w/v) to 0.5%(w/v), 0.15%(w/v) to 0.4%(w/v), 0.15%(w/v) to 0.3%(w/v), 0.15%(w/v) to 0.25%(w/v), or 0.2%(w/v));
(2) (2-1) mannitol at a concentration of about 5.5mM to about 55mM, about 5.5mM to about 41.25mM, about 5.5mM to about 27.5mM, about 5.5mM to about 22mM, about 5.5mM to about 16.5mM, about 5.5mM to about 14mM, about 8.25mM to about 55mM, about 8.25mM to about 41.25mM, about 8.25mM to about 27.5mM, about 8.25mM to about 22mM, about 8.25mM to about 16.5mM, about 8.25mM to about 14mM, or about 11mm (or 0.1%(w/v) to 1%(w/v), 0.1%(w/v) to 0.75%(w/v), 0.1%(w/v) to 0.5%(w/v), 0.1%(w/v) to 0.4%(w/v), 0.1%(w/v) to 0.3%(w/v), 0.1%(w/v) to 0.25%(w/v), 0.15%(w/v) to 1%(w/v), 0.15%(w/v) to 0.75%(w/v), 0.15%(w/v) to 0.5%(w/v), 0.15%(w/v) to 0.4%(w/v), 0.15%(w/v) to 0.3%(w/v), 0.15%(w/v) to 0.25%(w/v), or 0.2%(w/v)), and (2-2) glycine at a concentration of about 130mM to about 400mM, about 130mM to about 335mM, about 130mM to about 270mM, about 240mM to about 400mM, about 240mM to about 335mM, about 240mM to about 270mM, about 260mM to about 400mM, about 260mM to about 335mM, about 260mM to about 270mM, or about 266mM (or 1%(w/v) to 3%(w/v), 1%(w/v) to 2.5%(w/v), 1%(w/v) to 2%(w/v), 1.8%(w/v) to 3%(w/v), 1.8%(w/v) to 2.5%(w/v), 1.8%(w/v) to 2%(w/v), 2%(w/v) to 3%(w/v), 2%(w/v) to 2.5%(w/v), or 2%(w/v)); or
(3) (3-1) mannitol at a concentration of about 5.5mM to about 55mM, about 5.5mM to about 41.25mM, about 5.5mM to about 27.5mM, about 5.5mM to about 22mM, about 5.5mM to about 16.5mM, about 5.5mM to about 14mM, about 8.25mM to about 55mM, about 8.25mM to about 41.25mM, about 8.25mM to about 27.5mM, about 8.25mM to about 22mM, about 8.25mM to about 16.5mM, about 8.25mM to about 14mM, or about 11mM (or 0.1%(w/v) to 1%(w/v), 0.1%(w/v) to 0.75%(w/v), 0.1%(w/v) to 0.5%(w/v), 0.1%(w/v) to 0.4%(w/v), 0.1%(w/v) to 0.3%(w/v), 0.1%(w/v) to 0.25%(w/v), 0.15%(w/v) to 1%(w/v), 0.15%(w/v) to 0.75%(w/v), 0.15%(w/v) to 0.5%(w/v), 0.15%(w/v) to 0.4%(w/v), 0.15%(w/v) to 0.3%(w/v), 0.15%(w/v) to 0.25%(w/v), or 0.2%(w/v)), and (3-2) glycerol at a concentration of about 162mM to about 326mM, about 162mM to about 300mM, about 162mM to about 272mM, about 218mM to about 326mM, about 218mM to about 300mM, about 218mM to about 272mM, about 239mM to about 326mM, about 239mM to about 300mM, about 239mM to about 272mM, or about 239mM (v 1.5%(w/v) to 3%(w/v), 1.5%(w/v) to 2.75%(w/v), 1.5%(w/v) to 2.5%(w/v), 2%(w/v) to 3%(w/v), 2%(w/v) to 2.75%(w/v), 2%(w/v) to 2.5%(w/v), 2.2%(w/v) to 3%(w/v), 2.2%(w/v) to 2.75%(w/v), 2.2%(w/v) to 2.5%(w/v), or 2.2%(w/v)).

### Surfactant

The surfactant that can be contained in the pharmaceutical composition provided herein may be selected from all the pharmaceutically acceptable surfactants that can evenly disperse proteins in a liquid composition medium. The surfactant may be a non-ionic surfactant, particularly, polysorbates (e.g., polysorbate 20 (polyoxyethylene 20 sorbitan monolaurate), polysorbate 40 (polyethylene 20 sorbitan monopalmitate), polysorbate 60 (polyethylene 20 sorbitan monostearate), polysorbate 65 (polyethylene 20 sorbitan tristearate), polysorbate 80 (polyethylene 20 sorbitan monooleate), polysorbate 85 (polyethylene 20 sorbitan trioleate) (the number (20) following the polyoxyethylene means a total number of oxyethylene groups (-(CH2CH2O)-)), polyoxyethylene-polyoxypropylene block copolymers (poloxamers; e.g., Poloxamer 188 (Pluronic F-68), etc.). For instance, the surfactant may include polysorbate 20 or polysorbate 80, more specifically, polysorbate 20. More specifically, the surfactant may include polysorbate 20 and polysorbate 80 and may exclude at least one or all of the surfactants mentioned above.

The surfactant may be contained at a concentration of 0.001 to 5%(w/v), 0.001 to 3%(w/v), 0.001 to 1%(w/v), 0.001 to 0.5%(w/v), 0.001 to 0.3%(w/v), 0.001 to 0.2%(w/v), 0.001 to 0.1%(w/v), 0.001 to 0.05%(w/v), 0.001 to 0.02%(w/v), 0.001 to 0.01%(w/v), 0.005 to 5%(w/v), 0.005 to 3%(w/v), 0.005 to 1%(w/v), 0.005 to 0.5%(w/v), 0.005 to 0.3%(w/v), 0.005 to 0.2%(w/v), 0.005 to 0.1%(w/v), 0.005 to 0.05%(w/v), 0.005 to 0.02%(w/v), 0.005 to 0.01%(w/v), 0.01 to 5%(w/v), 0.01 to 3%(w/v), 0.01 to 1%(w/v), 0.01 to 0.5%(w/v), 0.01 to 0.3%(w/v), 0.01 to 0.2%(w/v), 0.01 to 0.1%(w/v), 0.01 to 0.05%(w/v), 0.01 to 0.02%(w/v), 0.05 to 5%(w/v), 0.05 to 3%(w/v), 0.05 to 1%(w/v), 0.05 to 0.5%(w/v), 0.05 to 0.3%(w/v), 0.05 to 0.2%(w/v), 0.05 to 0.1%(w/v), for example, about 0.01%(w/v) or about 0.1%(w/v), based on the total volume of the pharmaceutical composition.

### Other additives

The pharmaceutical composition provided herein may further include a pharmaceutically acceptable carrier and/or diluent. The pharmaceutically acceptable carrier and/or diluent may be one typically used in the art and include at least one selected from the group consisting of lactose, dextrose, sucrose, sorbitol, starch, acacia gum, calcium phosphate, alginate, gelatin, calcium silicate, microcrystalline cellulose, polyvinylpyrrolidone, cellulose, water (e.g., purified water), physiological saline, syrup, methyl cellulose, methylhydroxybenzoate, propylhydroxybenzoate, talc, magnesium stearate, and mineral oil, but with no limitations thereto.

The pharmaceutical composition provided herein may exclude at least one or all selected from the group consisting of:
- sodium salts (e.g., sodium chloride, sodium sulfate, etc.),
- potassium salts (e.g., potassium chloride, potassium hydroxide, etc.),
- ammonium salts (e.g., ammonium chloride, ammonium sulfate, ammonium carbonate, ammonium nitrate, etc.), and
- ethylenediaminetetraacetic acid (EDTA)

### pH

The pharmaceutical composition provided herein may have a pH of about 4 to about 7.5, about 4 to about 7, about 4 to about 6.5, about 4 to about 6, about 4 to about 5.5, about 4 to about 5, about 4.5 to about 7.5, about 4.5 to about 7, about 4.5 to about 6.5, about 4.5 to about 6, about 4.5 to about 5.5, about 4.5 to about 5, about 4.7 to about 7.5, about 4.7 to about 7, about 4.7 to about 6.5, about 4.7 to about 6, about 4.7 to about 5.5, about 4.7 to about 5, about 5.0 to about 7.5, about 5.0 to about 7, about 5.0 to about 6.5, about 5.0 to about 6, about 5.0 to about 5.5, about 5.3 to about 7.5, about 5.3 to about 7.2, about 5.3 to about 7, about 5.3 to about 6.8, about 5.3 to about 6.5, about 5.3 to about 5.5, about 5.5 to about 7.5, about 5.5 to about 7.2, about 5.5 to about 7, about 5.5 to about 6.8, about 5.5 to about 6.5, about 5.5 to about 6, about 6.0 to about 7.5, about 6.0 to about 7.2, about 6.0 to about 7, about 6.0 to about 6.8, about 6.0 to about 6.5, about 6.4 to about 7.5, about 6.4 to about 7.2, about 6.4 to about 7, about 6.4 to about 6.8, or about 6.4 to about 6.6, for example, about 4.0, about 4.7, about 5.0, about 5.2, or about 6.5.

### Stability

The pharmaceutical composition provided herein is characterized by its excellent stability. Herein, the term "excellent stability" or "stably maintained" means that the structure and/or physical, chemical, and/or biological properties of the protein in the composition are maintained during storage (for example, low protein aggregation rate, low protein degradation rate, low subvisible particle content(number), high main component content, low rate of charge alteration, etc.).

The aggregation rate means the proportion (degree) at which antibodies in the formulation aggregate to form high molecular weight products (HMW; aggregate).

The degradation rate means the proportion (degree) at which antibodies in the formulation degrade to form low molecular weight products (LMW; degradation products).

The subvisible particles refer to impurities included in the formulation, which may be particles with an average diameter of about 5 um or more.

The main component content refers to the content of the antibody in the monomer form (not aggregated or degraded) in the formulation.

The rate of charge alteration refers to the proportion (degree) at which the net charge on the surface of antibodies in the formulation is altered, forming acidic variants and/or basic variants.

The composition (liquid antibody formulation) provided herein contains antibodies (anti-TNFα antibody) at a high concentration (specifically, 80 mg/ml or more, 100 mg/ml or more, 120 mg/ml or more, or 150 mg/ml or more; the upper limit of 200 mg/ml), and exhibits "enhanced stability" (e.g., low protein aggregation rate, low protein degradation rate, low subvisible particle content (number), high main component content, low alteration rate, etc.), compared to the case of excluding, for a buffer, one or more selected from the group consisting of succinate, phosphate, acetate, and their pharmaceutically acceptable salts (e.g., succinate or pharmaceutically acceptable salts thereof), and/or for an excipient, solely mannitol, one or more selected from the group consisting of mannitol, glycerol, glycine, glucose, and glutamic acid, or one or more selected from the group consisting of mannitol, glycerol, and glycine, and/or for a surfactant, polysorbate 20.

Compared to cases where these components are included, can be "enhanced in stability" (for example, showing low protein aggregation rate, low protein degradation rate, low subvisible particle content (number), high main component content, low alteration rate, etc.), during: (1) storage at 5°C to 40°C (for example, 5°C, 25°C, and/or 40°C) for 1 to 8 weeks, or (2) under stress conditions (for example, agitation stress and/or freeze-thaw stress), or (3) after frozen storage (for example, storage for 3 months at -70°C conditions), where the content of acidic and/or basic variants is low, but is not limited to these conditions.

For instance, the "enhanced stability" means pertaining to showing (1) low protein aggregation rate, low protein degradation rate, low subvisible particle content (number), high main component content, and low alteration rate during storage at 5°C to 40°C (e.g., 5°C, 25°C, and/or 40°C) for 1 to 8 weeks, and/or (2) low protein aggregation rate, low protein degradation rate, low subvisible particle content (number), high main component content, and low alteration rate under stress conditions (e.g., agitation stress and/or freeze-thaw stress), and/or (3) low contents of acidic and/or basic variants after frozen storage (e.g., storage for 3 months at -70°C conditions), but with no limitations thereto, but with no limitations thereto, compared to the case of excluding, for a buffer, one or more selected from the group consisting of succinate, phosphate, acetate, and their pharmaceutically acceptable salts (e.g., succinate or pharmaceutically acceptable salts thereof), and/or for an excipient, solely mannitol, one or more selected from the group consisting of mannitol, glycerol, glycine, glucose, and glutamic acid, or one or more selected from the group consisting of mannitol, glycerol, and glycine, and/or for a surfactant, polysorbate 20.

### Osmotic pressure

The pharmaceutical composition provided herein may be isotonic with the body. Specifically, the liquid composition may have an osmolarity of about 200 mOsm/kg to about 550 mOsm/kg, about 200 mOsm/kg to about 500 mOsm/kg, about 200 mOsm/kg to about 400 mOsm/kg, about 250 mOsm/kg to about 400 mOsm/kg, about 200 mOsm/kg to about 350 mOsm/kg, about 250 mOsm/kg to about 350 mOsm/kg, about 200 to about 300 mOsm/kg, or about 250 to about 300 mOsnVkg.

### Route of Administration

The pharmaceutical composition provided herein may be administered orally or parenterally. For parenteral administration (e.g., injection), intravenous, subcutaneous, intramuscular, intraperitoneal, intradermal, topical, intranasal, intrapulmonary, intrarectal, and intratumoral routes may be taken. For oral administration, since the protein can be digested, the active protein ingredient can be coated or formulated to be protected from degradation in the stomach.

### Advantageous Effects

Provided herein is a formulation that contains antibodies at relatively high concentrations (specifically, 80 mg/ml or more, 100 mg/ml or more, 120 mg/ml or more, or 150 mg/ml or more) while maintaining excellent stability.

### Mode for Invention

A better understanding of the present disclosure may be obtained via the following examples which are set forth to illustrate, but are not to be construed as limiting the present disclosure.

### EXAMPLE 1. Preparation of Antibody and Formulation

For selecting formulations with excellent chemical and physical stability for antibodies, the following candidate formulations were prepared.

For use as an antibody, adalimumab (Adalimumab; anti-TNFα monoclonal antibody; CAS Registry Number: 331731-18-1; DrugBank Accession Number DB00051) was constructed in house.

Adalimumab light-chain amino acid sequence (SEQ ID NO: 1):

DIQMTQSPSSLSASVGDRVTITCRASQGIRNYLAWYQQKPGKAPKLLIYAAS
TLQSGVPSRFSGSGSGTDFTLTISSLQPEDVATYYCQRYNRAPYTFGQGTKVEIKRTV
AAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC

Adalimumab heavy-chain amino acid sequence (SEQ ID NO: 2):

EVQLVESGGGLVQPGRSLRLSCAASGFTFDDYAMHWVRQAPGKGLEWVSA
ITWNSGHIDYADSVEGRFTISRDNAKNSLYLQMNSLRAEDTAVYYCAKVSYLSTAS
SLDYWGQGTLVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDKKV
EPKSCDKTHTCPPCPAPELLGGPSVFLFPPKPKDTLMISRTPEVTCVVVDVSHEDPEV
KFNWYVDGVEVHNAKTKPREEQYNSTYRVVSVLTVLHQDWLNGKEYKCKVSNK
ALPAPIEKTISKAKGQPREPQVYTLPPSRDELTKNQVSLTCLVKGFYPSDIAVEWESN
GQPENNYKTTPPVLDSDGSFFLYSKLTVDKSRWQQGNVFSCSVMHEALHNHYTQK
SLSLSPGK

Selection was made of four buffers 10-20 mM histidine, phosphate, succinate, and acetate; and five excipients 10-300 mM glucose, glycerol, mannitol, glycine, and ethanol. pH was adjusted into a range of 4-6.5. A surfactant was selected through screening for Polysorbate 20 (PS20), Polysorbate 80 (PS80), and Pluronic^{®} F68 (poloxamer 188; F-68) at concentrations of 0.01%-0.1%. The selected formulation conditions were applied to adalimumab through buffer exchange using dialysis or centrifugation.

The concentration of adalimumab in the formulation was adjusted within the range of 1 to 200 mg/mL.

Buffers, excipient, and surfactant conditions for adalimumab formulations are summarized in Table 1, below.

**TABLE 1**

| **Buffer** | **Excipient** | **Surfactant** |
|---|---|---|
| Acetate | Mannitol | Polysorbate 20 (PS20) |
| Succinic Acid | Glycine | Polysorbate 80 (PS80) |
| Sodium Phosphate | Glycerol | Poloxamer 188 (F-68) |
| L-Histidine | Dextrose (Glucose) | |
| | Ethanol | |

### REFERENCE EXAMPLE 1. Physical Property Test of Antibody under Stress Conditions

1.1. Adalimumab was analyzed for structural change using differential scanning fluorimetry (DSF) when subjected to a temperature change from 20°C to 95°C under the condition containing 1 mg/ml adalimumab.

1.2. The quantitative change of adalimumab monomer was measured using size exclusion high-performance liquid chromatography (SE-HPLC) while concentrating adalimumab up to 200 mg/mL in the formulation conditions.

1.3. During storage for 8 weeks at 5°C, 25°C, or 40°C under the condition containing 100 mg/ml adalimumab, measurement was made of the quantitative change of adalimumab monomer using SE-HPLC and the number of subvisible particles using Micro-Flow Imaging (MFI).

1.4. After adding PS20 to the condition containing adalimumab at concentrations up to ~200 mg/mL, agitation stress at 600 rpm for 3 days or 5 cycles of freeze-thaw stress were applied. Then, the concentration of adalimumab was diluted to 1 mg/mL before the quantitative change of adalimumab monomer was measured using SE-HPLC.

1.5. After storage of each formulation containing adalimumab at a concentration of 100 mg/mL at -70°C for 3 months, the adalimumab concentration was diluted to 1 mg/mL under which the change in charge variants was measured using cation exchange high-performance liquid chromatography (CEX-HPLC).

### REFERENCE EXAMPLE 2. Testing Method for Physical Properties of Antibodies

The chemical and physical stability of adalimumab within the formulations prepared in Reference Example 1 was evaluated.

### 2.1. Thermal Stability Evaluation

To evaluate thermal stability, 8.8 µL of buffer-exchanged adalimumab solution at a concentration of 1 mg/mL was analyzed for its melting temperature (Tm, the protein's melting point) using differential scanning fluorimetry (DSF) with the UNCLE device from UNChained Labs. The structural changes in adalimumab induced by heat were observed by ramping the temperature from 20°C to 95°C at a rate of 60°C per hour using DSF.

### 2.2. Soluble aggregate assessment

Formulation stability was evaluated by measuring degradation and aggregation characteristics of antibodies in the formulations through size exclusion high-performance liquid chromatography (SE-HPLC).

Specifically, adalimumab in each formulation was diluted to 1 mg/mL with a mobile phase containing 0.001% PS80 (50 mM sodium phosphate, 0.15 M sodium chloride, pH 6.8). Twenty µL of the diluted adalimumab solution was analyzed using an Agilent (1100 or 1260) instrument and TSK gel G3000SWXL column to measure the changes in the amount of adalimumab monomer (main peak; %), high molecular weight components (HMW; %), and low molecular weight components (LMW; %). High molecular weight (HMW; %) = {area of HMW / area of (HMW + monomer + LMW)} * 100) Low molecular weight (LMW; %) = {area of LMW / area of (HMW + monomer + LMW)} * 100) Monomer (main peak; %) = {area of main peak / area of (HMW + main peak + LMW)} * 100)\

### 2.3. Subvisible Particle analysis

Subvisible particles in the formulations were identified using micro-flow imaging (MFI) or FlowCAM.

Specifically, 0.5 mL of the adalimumab stock solution or a 1:1 diluted solution was analyzed using the FlowCAM from Fluid Imaging Technologies or the MFI5200 instrument from ProteinSimple to measure the number of subvisible particles (>5µm (over 5µm), >10µm (over 10µm), or >25µm (over 25µm)) in the formulations containing adalimumab.

### 2.4. Charge variants assessment (long-term freezing stability evaluation)

Formulation stability was evaluated by measuring the surface charge heterogeneity of antibodies within the formulation using cation exchange high-performance liquid chromatography (CEX-HPLC).

Specifically, adalimumab in each formulation was diluted to 1 mg/mL with mobile phase A (10 mM Sodium Phosphate, pH 7.0). Twenty µL of the diluted adalimumab solution was applied to a Propac WCX-10 column using an Agilent (1100 or 1260) instrument. The changes in the amount of the main peak (main peak; %), acidic variants (Acidic Variants, Pre-Peaks; %), and basic variants (Basic Variants, Post-Peaks; %) of adalimumab that eluted due to the increase in salt concentration through a gradient of mobile phase B (10 mM Sodium Phosphate, 0.5 M Sodium Chloride, pH 7.0) were measured as follows: - Main peak (Main peak; %) = {area of Main Peak/area of (Pre-Peaks + Main Peak + Post-Peaks)]*100) - Acidic Variants (Pre-Peaks; %) = {area of Pre-Peaks/area of (Pre-Peaks + Main Peak + Post-Peaks)] * 100) - Basic Variants (Post-Peaks; %) = {area of Post-Peaks/area of (Pre-Peaks + Main Peak + Post-Peaks)] * 100)

### EXAMPLE 2. Selection of Buffer and Excipient (Thermal Stability Evaluation)

Adalimumab was measured for thermal stability in 30 different formulation conditions prepared according to Example 1 (see Table 2 below).

Three types of buffers (20 mM each of histidine, phosphate and succinate), five types of excipients (200 mM each of glucose, glycerol and mannitol, 100 mM glycine, 6% glycine), and two pH levels (5 and 6.5) were selected, resulting in a total of 30 formulations. For a control formulation, adalimumab constructed in house was applied to the condition of 42 mg/ml mannitol and 0.1% PS80, which is the same as in the Humira (Humira; AbbVie) formulation.

Specifically, 13 mg of the in-house constructed adalimumab (approximately 130 mg/ml adalimumab in a 42 mg/ml mannitol solution) was placed in an Amicon Ultra centrifugal filter and diluted 10-50 times with each of the solutions of the 30 formulations and the control group formulation. Centrifugation was performed at 4000g until the volume was reduced to one-tenth. These processes were repeated three times to complete the formulations. The same formulation solutions were used to dilute adalimumab to 1.0 mg/ml, and thermal stability was evaluated.

The thermal stability evaluation was conducted by measuring Tm with reference to the method in Reference Example 2.1.

The conditions and average Tm values of the 30 types of formulations prepared and tested in this Example are summarized in Table 2 below.

**TABLE 2**

| **Form. No.** | **Buffer** | **pH** | **Excipient** | **Surfactant** | **Average Tm** |
|---|---|---|---|---|---|
| 1 | 20 mM Histidine | 5 | 200 mM Mannitol | - | 65.5 |
| 2 | 20 mM Histidine | 5 | 100 mM Glycine | - | 68.7 |
| 3 | 20 mM Histidine | 5 | 200 mM Glucose | - | 65.5 |
| 4 | 20 mM Histidine | 5 | 200 mM Glycerol | - | 68.3 |
| 5 | 20 mM Histidine | 5 | 6 % Ethanol | - | 64.4 |
| 6 | 20 mM Histidine | 6.5 | 200 mM Mannitol | - | 64.6 |
| 7 | 20 mM Histidine | 6.5 | 100 mM Glycine | - | 64.4 |
| 8 | 20 mM Histidine | 6.5 | 200 mM Glucose | - | 65.7 |
| 9 | 20 mM Histidine | 6.5 | 200 mM Glycerol | - | 70.9 |
| 10 | 20 mM Histidine | 6.5 | 6 % Ethanol | - | 68.1 |
| 11 | 20 mM Phosphate | 5 | 200 mM Mannitol | - | 71 |
| 12 | 20 mM Phosphate | 5 | 100 mM Glycine | - | 70.4 |
| 13 | 20 mM Phosphate | 5 | 200 mM Glucose | - | 70.2 |
| 14 | 20 mM Phosphate | 5 | 200 mM Glycerol | - | 70 |
| 15 | 20 mM Phosphate | 5 | 6 % Ethanol | - | 65.4 |
| 16 | 20 mM Phosphate | 6.5 | 200 mM Mannitol | - | 70.1 |
| 17 | 20 mM Phosphate | 6.5 | 100 mM Glycine | - | 70.4 |
| 18 | 20 mM Phosphate | 6.5 | 200 mM Glucose | - | 70.5 |
| 19 | 20 mM Phosphate | 6.5 | 200 mM Glycerol | - | 70.2 |
| 20 | 20 mM Phosphate | 6.5 | 6 % Ethanol | - | 65.7 |
| 21 | 20 mM Succinate | 5 | 200 mM Mannitol | - | 70.7 |
| 22 | 20 mM Succinate | 5 | 100 mM Glycine | - | 70.2 |
| 23 | 20 mM Succinate | 5 | 200 mM Glucose | - | 69.3 |
| 24 | 20 mM Succinate | 5 | 200 mM Glycerol | - | 68.7 |
| 25 | 20 mM Succinate | 5 | 6 % Ethanol | - | 62.1 |
| 26 | 20 mM Succinate | 6.5 | 200 mM Mannitol | - | 65.2 |
| 27 | 20 mM Succinate | 6.5 | 100 mM Glycine | - | 70.9 |
| 28 | 20 mM Succinate | 6.5 | 200 mM Glucose | - | 70.7 |
| 29 | 20 mM Succinate | 6.5 | 200 mM Glycerol | - | 70.6 |
| 30 | 20 mM Succinate | 6.5 | 6 % Ethanol | - | 65.1 |

As shown in Table 2, the thermal stability of the test groups using phosphate or succinate as buffers was found to be superior to that of the test groups using histidine. Additionally, the thermal stability of the test groups using ethanol as an excipient (Excipient) was found to be lower compared to other test groups.

### EXAMPLE 3. Assessment of Physical Properties of Buffer and Excipient (Soluble Aggregate)

Formulations were prepared by combining buffer and excipient conditions that were identified as excellent in thermal stability in Example 2, and the solubility (soluble aggregate) of adalimumab was tested.

More specifically, two types of buffers (20 mM phosphate or succinate), three types of excipients (glycerol, mannitol, and glycine), and two pH levels (5 and 6.5) were selected to prepare a total of 12 formulations (see Table 3 below). As a control, a formulation identical to Humira (Humira; AbbVie) with 42 mg/ml mannitol, 0.1% PS80, pH 5.2 was prepared for comparative evaluation.

In brief, 155 mg of in-house constructed adalimumab antibody (about 120 mg/ml adalimumab, 42 mg/ml mannitol solution) was placed in an Amicon Ultra centrifugal filter and centrifuged at 4000g until the concentration reached 200 mg/mL. Subsequently, the solution was diluted 10 times with the formulation solutions of the 12 conditions containing buffer and excipient and with the control formulation, and centrifuged at 4000g until the volume was reduced to one-tenth. These processes were repeated three times, and additional centrifugation was performed to obtain the desired composition of liquid adalimumab formulation at a concentration of 200 mg/mL.

For each obtained formulation, size exclusion chromatography high-performance liquid chromatography (SEC-HPLC) was performed with reference to the method in Reference Example 2.2, and the content of the main component (main peak), high molecular weight, and low molecular weight were measured.

The conditions and SEC-HPLC results of the 12 types of formulations manufactured and tested in this Example are summarized in Table 3 below.

**TABLE 3**

| **Form. No.** | **Buffer** | **pH** | **Excipient** | **Surfactant** | **Main Peak Area % [%]** | **EMW Area % [%]** | **LMW Area %[%]** |
|---|---|---|---|---|---|---|---|
| 1 | 20 mM Succinate | 5 | 75mM mannitol | - | 99.27 | 0.6 | 0.13 |
| 2 | 20 mM Succinate | 5 | 100 mM Glycine, 100 mM mannitol | - | 99.26 | 0.57 | 0.17 |
| 3 | 20 mM Succinate | 5 | 150 mM Glycerol, 25mM mannitol | - | 99.22 | 0.62 | 0.16 |
| 4 | 20 mM Succinate | 6.5 | 75mM mannitol, 150mM mannitol | - | 99.18 | 0.69 | 0.13 |
| 5 | 20 mM Succinate | 6.5 | 100 mM Glycine, 25mM mannitol | - | 99.23 | 1.01 | 0.13 |
| 6 | 20 mM Succinate | 6.5 | 150 mM Glycerol, 100mM mannitol | - | 99.15 | 0.64 | 0.13 |
| 7 | 20 mM Phosphate | 5 | 150mM mannitol | - | 99.28 | 0.56 | 0.16 |
| 8 | 20 mM Phosphate | 5 | 100 mM Glycine, 100 mM mannitol | - | 99.29 | 0.54 | 0.17 |
| 9 | 20 mM Phosphate | 5 | 150 mM Glycerol, 25mM mannitol | - | 99.21 | 0.61 | 0.18 |
| 10 | 20 mM Phosphate | 6.5 | 75mM mannitol | - | 99.13 | 0.68 | 0.19 |
| 11 | 20 mM Phosphate | 6.5 | 25mM Glycine, 25mM mannitol | - | 99.18 | 0.65 | 0.17 |
| 12 | 20 mM Phosphate | 6.5 | 100mM Glycerol, 100mM mannitol | - | 99.12 | 0.72 | 0.16 |
| Control | No buffer | 5.2 | 42mg/ml(231mM) Mannitol | 0.1% PS80 | 98.94 | 0.87 | 0.19 |

As shown in Table 3, all 12 tested formulations exhibited higher purity (main peak%) and equal or lower HMW% (high molecular weight component content) and LMW% (low molecular weight component content) compared to the control group, indicating that all 12 formulations have stability equal to or better than that in the control group. Particularly, in test groups using phosphate and succinate as buffers, higher purity (main peak%) and lower HMW% were observed at pH 5 than at pH 6.5.

### EXAMPLE 4. Selection of Surfactant (Assessment of Subvisible Particles)

The adalimumab constructed in-house was prepared at a concentration of 101 mg/mL (42 mg/mL mannitol), and a surfactant was added to achieve a final concentration of 100 mg/mL adalimumab.

Polysorbate 20 (PS20), Polysorbate 80 (PS80), and Pluronic^{®} F68 (F-68) were used as surfactants. Formulations were prepared by adding a 1% (w/v) solution to achieve 0.01% (w/v) for PS20 and PS80 and a 10% (w/v) solution to achieve 0.1% (w/v) for F-68.

After agitating the prepared formulations at room temperature at 1000 rpm for 4 hours, the number of subvisible particles (particles with an average diameter of 5 µm or more) in the formulations was measured using FlowCAM with reference to the method in Reference Example 2.3.

The measurements are presented in Table 4:

**TABLE 4**

| Particle size | >5µm | >10 | >25µm |
|---|---|---|---|
| Water | 0 | 0 | 0 |
| No Surfactant | 631 | 321 | 65 |
| **0.01% PS20** | **12** | **3** | **0** |
| 0.01% PS80 | 15 | 3 | 0 |
| 0.1 % F-68 | 58 | 15 | 0 |

As shown in Table 4, the test groups using PS20 and PS80 as surfactants had lower subvisible particle content, compared to the test group using F-68, with the lowest subvisible particle content in the test group using PS20.

### EXAMPLE 5. Evaluation for Stability of Selected Formulations under Stress Conditions

### 5.1. Preparation of Formulations

Based on the results of Example 4, 12 formulations containing 0.01% (w/v) PS20 as a surfactant were prepared.

Briefly, three types of buffers (10 mM phosphate, succinate, and histidine), five types of excipients (mannitol, glycine, glycerol, glucose, and glutamic acid), and five pH points between 4 and 6.2 were selected. Additionally, 0.01% (w/v) PS20 was added as a surfactant to prepare a total of 12 liquid formulations (adalimumab concentration: 100 mg/ml). As a control, a formulation identical to Humira (Humira; AbbVie) with 42 mg/ml mannitol, 0.1% PS80, pH 5.2 was prepared for comparative evaluation (adalimumab concentration: 100 mg/ml).

Specifically, the in-house constructed adalimumab (about 120 mg/ml adalimumab, 42 mg/ml mannitol solution) was added and thoroughly mixed at a dilution ratio of 1:1000 with 10% PS20. Dialysis was performed using solutions of buffer and excipient in the PS20-added adalimumab solution to ensure more than 10,000-fold exchange, and dilution or centrifugation was performed in each formulation condition to achieve an adalimumab concentration of 100 mg/ml, thus preparing high-concentration adalimumab formulations.

The conditions of the 12 prepared formulations are summarized in Table 5 below:

**TABLE 5**

| Form. No. | Buffer | pH | Excipient | Surfactant | Osmoral ity | Viscosity at 25°C (cP) |
|---|---|---|---|---|---|---|
| 1 | 10 mM succinate | 4 | 11mM(0.2%) Mannitol, 266mM(2%) Glycine | 0.01% PS20 | 313 | 3.31 |
| 2 | 10 mM succinate | 4.7 | 11mM(0.2%) Mannitol, 239mM(2.2%) Glycerol | 0.01% PS20 | 297 | 3.62 |
| 3 | 10 mM succinate | 4.7 | 11mM(0.2%) Mannitol, 278mM(5%) Glucose | 0.01% PS20 | 343 | 4.32 |
| 4 | 10 mM succinate | 5.2 | 11mM(0.2%) Mannitol, 266mM(2%) Glycine | 0.01% PS20 | 320 | 4.84 |
| 5 | 10 mM succinate | 5.2 | 11mM(0.2%) Mannitol, 239mM(2.2%) Glycerol | 0.01% PS20 | 299 | 4.1 |
| 6 | 10 mM succinate | 5.2 | 11mM(0.2%) Mannitol, 278mM(5%) Glucose | 0.01% PS20 | 348 | 5.23 |
| 7 | 10mM phosphate | 5.7 | 11mM(0.2%) Mannitol, 266mM(2%) Glycine | 0.01% PS20 | 321 | 3.71 |
| 8 | 10mM phosphate | 5.7 | 11mM(0.2%) Mannitol, 239mM(2.2%) Glycerol | 0.01% PS20 | 317 | 3.84 |
| 9 | 10mM phosphate | 5.7 | 11mM(0.2%) Mannitol, 278mM(5%) | 0.01% PS20 | 382 | 4.36 |
| | | | Glucose | | | |
| 10 | 10mM phosphate | 6.2 | 11mM(0.2%) Mannitol, 266mM(2%) Glycine | 0.01% PS20 | 310 | 3.01 |
| 11 | 10mM phosphate | 6.2 | 11mM(0.2%) Mannitol, 278mM(5%) Glucose | 0.01% PS20 | 370 | 4.87 |
| Control | No buffer | 5.2 | 42 mg/mL mannitol | 0.1% PS80 | 277 | 4.18 |

(In Table 5, "%": %(w/v); osmolarity (mmol/kg): measured at time zero using a Model 2020 Freezing Point Osmometer (Advanced Instruments) following the manufacturer's method; viscosity: measured at time zero at 25°C by injecting 130 µl of all samples into a glass syringe of an m-VROC Rheometer (Rheosense) at a flow rate of 150 µl/min)

### 5.2. Formulation Stability Test Depending on Temperature (SE-HPLC)

The 12 formulations and the control formulation prepared in Example 5.1 was tested for stability according to storage temperature and duration.

In brief, the prepared formulations were stored at typical storage conditions of 5°C and 25°C for 0 to 8 weeks, and at the stringent conditions of 40°C for 0 to 4 weeks. After storage, size exclusion high-performance liquid chromatography (SE-HPLC) was performed with reference to the method in Reference Example 2.2 to measure the content of the main component (main peak %) and high molecular weight content (HMW %).

The measurements are presented in Tables 6 to 11:

**TABLE 6**

| Main Component Content (main peak %) According to Storage Duration at 5°C | | | | | |
|---|---|---|---|---|---|
| Form. No. | 0W | 1W | 2W | 4W | 8W |
| 1 | 97.7 | 97.8 | 98 | 97.7 | 97.7 |
| 2 | 97.3 | 97.3 | 97.6 | 97.2 | 97.2 |
| 3 | 97.3 | 97.5 | 97.6 | 97.3 | 97.3 |
| 4 | 97.4 | 97.4 | 97.6 | 97.4 | 97.3 |
| 5 | 97 | 97.2 | 97.3 | 97 | 97 |
| 6 | 97 | 97.1 | 97.3 | 96.9 | 96.9 |
| 7 | 97 | 97.1 | 97.3 | 97 | 96.9 |
| 8 | 96 | 96.2 | 96.4 | 96 | 96 |
| 9 | 96.4 | 96.5 | 96.7 | 96.4 | 96.3 |
| 10 | 96.9 | 97.1 | 97.2 | 96.9 | 96.7 |
| 11 | 95.9 | 96.1 | 96.4 | 96 | 95.9 |
| Control | 95.1 | 95.6 | 95.8 | 95.3 | 95.3 |

**TABLE7**

| Main Component Content (main peak %) According to Storage Duration at 25°C | | | | | |
|---|---|---|---|---|---|
| Form. No. | 0W | 1W | 2W | 4W | 8W |
| 1 | 97.7 | 97.8 | 97.9 | 97.4 | 97 |
| 2 | 97.3 | 97.2 | 97.3 | 96.6 | 96.2 |
| 3 | 97.3 | 97.3 | 97.3 | 96.7 | 96.2 |
| 4 | 97.4 | 97.2 | 97.2 | 96.5 | 95.8 |
| 5 | 97 | 96.8 | 96.8 | 96.2 | 95.7 |
| 6 | 97 | 96.8 | 96.7 | 96 | 95.2 |
| 7 | 97 | 96.8 | 96.7 | 95.7 | 94.4 |
| 8 | 96 | 95.8 | 95.8 | 94.9 | 94.3 |
| 9 | 96.4 | 96.1 | 96 | 95 | 93.3 |
| 10 | 96.9 | 96.6 | 96.5 | 95.3 | 93.6 |
| 11 | 95.9 | 95.6 | 95.4 | 94 | 91 |
| Control | 95.1 | 95.2 | 95.1 | 94.1 | 93.6 |

**TABLE8**

| Main Component Content (main peak %) According to Storage Duration at 40°C | | | | |
|---|---|---|---|---|
| Form. No. | 0W | 1W | 2W | 4W |
| 1 | 97.7 | 97.4 | 96.9 | 93.3 |
| 2 | 97.3 | 96.4 | 95.8 | 93.5 |
| 3 | 97.3 | 96.2 | 94.3 | 86.1 |
| 4 | 97.4 | 96 | 95 | 91.9 |
| 5 | 97 | 95.8 | 94.9 | 92.6 |
| 6 | 97 | 95.2 | 92.3 | 82.1 |
| 7 | 97 | 95.3 | 93.5 | 88.1 |
| 8 | 96 | 94.4 | 93.3 | 91 |
| 9 | 96.4 | 93.1 | 86.7 | 72.3 |
| 10 | 96.9 | 94.9 | 92.6 | 85.5 |
| 11 | 95.9 | 90.8 | 81.2 | 64.4 |
| Control | 95.1 | 94 | 92.9 | 90.8 |

**TABLE 9**

| High-Molecular Weight Content (HMW %) According to Storage Duration at 5°C | | | | | |
|---|---|---|---|---|---|
| Form. No. | 0W | 1W | 2W | 4W | 8W |
| 1 | 2.2 | 2.1 | 1.9 | 2.1 | 2.1 |
| 2 | 2.6 | 2.6 | 2.3 | 2.6 | 2.6 |
| 3 | 2.6 | 2.5 | 2.3 | 2.5 | 2.6 |
| 4 | 2.6 | 2.5 | 2.2 | 2.5 | 2.5 |
| 5 | 2.9 | 2.8 | 2.5 | 2.8 | 2.9 |
| 6 | 2.9 | 2.8 | 2.6 | 2.9 | 2.9 |
| 7 | 2.9 | 2.8 | 2.6 | 2.9 | 3 |
| 8 | 3.9 | 3.7 | 3.5 | 3.8 | 3.9 |
| 9 | 3.5 | 3.4 | 3.1 | 3.5 | 3.5 |
| 10 | 3 | 2.8 | 2.7 | 3 | 3.1 |
| 11 | 4 | 3.8 | 3.5 | 3.9 | 4 |
| Control | 4.8 | 4.3 | 4 | 4.5 | 4.5 |

**TABLE 10**

| High-Molecular Weight Content (HMW %) According to Storage Duration at 25°C | | | | | |
|---|---|---|---|---|---|
| Form. No. | 0W | 1W | 2W | 4W | 8W |
| 1 | 2.2 | 2 | 1.8 | 2.2 | 2.3 |
| 2 | 2.6 | 2.7 | 2.5 | 3.1 | 3.4 |
| 3 | 2.6 | 2.6 | 2.5 | 3 | 3.4 |
| 4 | 2.6 | 2.7 | 2.6 | 3.2 | 3.7 |
| 5 | 2.9 | 3 | 3 | 3.5 | 4 |
| 6 | 2.9 | 3.1 | 3 | 3.7 | 4.5 |
| 7 | 2.9 | 3.1 | 3.1 | 4 | 5.2 |
| 8 | 3.9 | 4.1 | 4 | 4.9 | 5.4 |
| 9 | 3.5 | 3.7 | 3.8 | 4.7 | 6.3 |
| 10 | 3 | 3.2 | 3.2 | 4.4 | 6 |
| 11 | 4 | 4.2 | 4.4 | 5.8 | 8.6 |
| Control | 4.8 | 4.7 | 4.7 | 5.6 | 6.1 |

**TABLE 11**

| High-Molecular Weight Content (HMW %) According to Storage Duration at 40°C | | | | |
|---|---|---|---|---|
| Form. No. | 0W | 1W | 2W | 4W |
| 1 | 2.2 | 1.9 | 1.6 | 2.4 |
| 2 | 2.6 | 3.2 | 3.5 | 5.1 |
| 3 | 2.6 | 3.4 | 5 | 12.2 |
| 4 | 2.6 | 3.6 | 4.2 | 6.5 |
| 5 | 2.9 | 3.9 | 4.4 | 6.1 |
| 6 | 2.9 | 4.4 | 7 | 16.6 |
| 7 | 2.9 | 4.3 | 5.8 | 10.4 |
| 8 | 3.9 | 5.2 | 6.1 | 7.9 |
| 9 | 3.5 | 6.5 | 12.6 | 26.2 |
| 10 | 3 | 4.6 | 6.6 | 12.9 |
| 11 | 4 | 8.8 | 18 | 33.8 |
| Control | 4.8 | 5.7 | 6.5 | 8 |

In Tables 6 to 11, it was observed that the test formulations, especially formulations 1, 2, 4, and 5, compared to the control group formulation, showed higher main component content and lower high molecular weight content initially (at 0 weeks), and maintained higher main component content and/or lower high molecular weight content throughout all testing intervals during long-term storage (~8 weeks) under all temperature conditions. This indicates improved initial formulation stability and storage stability compared to the control group formulation.

### 5.3. Test for Formulation Stability Over Storage Duration (MFI Analysis)

The stability of the 12 formulations and the control group formulation prepared in Example 5.1 was tested over the storage period.

In brief, the prepared formulations were stored at 5°C, 25°C, or 40°C for 0 to 8 weeks. After storage, Micro-Flow Imaging (MFI) analysis was performed with reference to the method in Reference Example 2.3 to measure subvisible particles.

The measurements are presented in Table 12:

**TABLE 12**

| No. | 0 Weeks | | 8 Weeks (5°C) | | 8 Weeks (25°C) | | 8 Weeks (40°C) | |
|---|---|---|---|---|---|---|---|---|
| | >10 µm | >25 µm | >10 µm | >25 µm | >10 µm | >25 µm | >10 µm | >25 µm |
| 1 | 173 | 18 | 82 | 12 | 9 | 0 | 127 | 9 |
| 2 | 61 | 0 | 70 | 0 | 112 | 6 | 153 | 0 |
| 3 | 533 | 55 | 3 | 0 | 245 | 33 | 388 | 15 |
| 4 | 136 | 3 | 3 | 0 | 9 | 0 | 130 | 12 |
| 5 | 40 | 6 | 48 | 3 | 45 | 0 | 61 | 0 |
| 6 | 209 | 27 | 12 | 3 | 103 | 9 | 12 | 0 |
| 7 | 91 | 12 | 21 | 3 | 91 | 6 | 976 | 87 |
| 8 | 407 | 31 | 158 | 21 | 203 | 15 | 439 | 61 |
| 9 | 236 | 27 | 158 | 18 | 97 | 9 | 139 | 6 |
| 10 | 512 | 59 | 309 | 45 | 279 | 30 | 427 | 24 |
| 11 | 470 | 24 | 82 | 12 | 267 | 39 | 473 | 18 |
| Control | 297 | 3 | 100 | 21 | 1354 | 58 | 248 | 24 |

As seen in Table 12, many of the 12 tested formulations exhibited a lower number of subvisible particles compared to the control under the tested storage durations and temperature conditions. Particularly, formulations containing succinate buffer and either mannitol and glycine or mannitol and glycerol (formulations 1, 2, 4, and 5) showed a relatively lower number of subvisible particles, demonstrating their excellent stability.

### EXAMPLE 6. Stability Evaluation of Formulations Containing Mannitol, Glycerol, and Glycine under Stress Conditions

### 6.1. Preparation of Formulations

Based on the results of Example 5, 12 formulations containing various combinations of mannitol, glycerol, and glycine as excipients were prepared.

Briefly, two types of buffers (10 mM phosphate, succinate), three types of excipients (glycerol, mannitol, glycine), and two types of surfactants (0.1% (w/v) PS20, 0.01% (w/v) PS20) were selected, with pH fixed at 5, to prepare a total of 12 pale yellow liquid formulations. As a control, a formulation identical to Humira (Humira; AbbVie) with 42 mg/ml mannitol, 0.1% PS80, pH 5.2 was prepared for comparative evaluation.

Specifically, 855 mg of the in-house constructed adalimumab was placed in an Amicon Ultra centrifugal filter and centrifuged at 4000g until the concentration reached 200 mg/mL. The antibody was then diluted 5-fold with the solutions composed of the buffer and excipient in each formulation condition and centrifuged at 4000g until the volume was reduced to one-fifth. These processes were repeated three times, and PS20 was added to achieve concentrations of 0.01% and 0.1%, thus preparing high-concentration (~200 mg/mL) adalimumab formulations.

The conditions of the 12 prepared formulations are summarized in Table 13 below:

**TABLE 13**

| Form. No. | Buffer | pH | Excipient | Surfactant |
|---|---|---|---|---|
| 1 | 10 mM Succinate | 5 | 150 mM Glycerol, 50mM mannitol | 0.1% PS20 |
| 2 | 10 mM Succinate | 5 | 100 mM Glycine, 50mM mannitol | 0.1% PS20 |
| 3 | 10 mM Succinate | 5 | 250mM mannitol | 0.1% PS20 |
| 4 | 10 mM Succinate | 5 | 150 mM Glycerol, 50mM mannitol | 0.01% PS20 |
| 5 | 10 mM Succinate | 5 | 100 mM Glycine, 50mM mannitol | 0.01% PS20 |
| 6 | 10 mM Succinate | 5 | 250mM mannitol | 0.01% PS20 |
| 7 | 10 mM Acetate | 5 | 150 mM Glycerol, 50mM mannitol | 0.1% PS20 |
| 8 | 10 mM Acetate | 5 | 100 mM Glycine, 50mM mannitol | 0.1% PS20 |
| 9 | 10 mM Acetate | 5 | 250mM mannitol | 0.1% PS20 |
| 10 | 10 mM Acetate | 5 | 150 mM Glycerol, 50mM mannitol | 0.01% PS20 |
| 11 | 10 mM Acetate | 5 | 100 mM Glycine, 50mM mannitol | 0.01% PS20 |
| 12 | 10 mM Acetate | 5 | 250mM mannitol | 0.01% PS20 |
| Control | No buffer | 5.2 | 42mg/ml(231mM) Mannitol | 0.1% PS80 |

### 6.2. Test for Formulation Stability under Stress Conditions (SE-HPLC)

The 12 formulations and the control group formulation prepared in Example 6.1 were tested for stability under stress conditions.

In brief, stress was applied to the prepared formulations, followed by performing size exclusion high-performance liquid chromatography (SE-HPLC) with reference to the method in Reference Example 2.2 to measure the content of the main component (main peak %), high molecular weight content (HMW %), and low molecular weight content (LMW %).

The stress was applied either as agitation stress or freeze-thaw stress. Agitation stress was induced by stirring the prepared formulations at room temperature (25°C) at 600 rpm for 3 days, followed by storage at 2-8°C for 1-2 days until analysis. Freeze-thaw stress involved repeating the process of freezing at -75°C for at least 1 hour and then thawing at room temperature (25°C) a total of 5 times, followed by storage at 2-8°C for 1-2 days until analysis.

For comparison, the main component content (main peak %), high molecular weight content (HMW %), and low molecular weight content (LMW %) were also measured under normal conditions (control; no separate stress applied, and stored at 2-8°C for 1-2 days after formulation preparation).

The measurements are presented in Tables 14 to 16:

**TABLE 14**

| Main Component Content (main peak %) | | | |
|---|---|---|---|
| Form. No. | Control | Agitation | Freeze-thaw |
| 1 | 99.1 | 98.9 | 98.9 |
| 2 | 99.1 | 99 | 99.1 |
| 3 | 99 | 99 | 99 |
| 4 | 99.1 | 99 | 99.1 |
| 5 | 99 | 99.1 | 99.2 |
| 6 | 98.9 | 98.9 | 99 |
| 7 | 98.9 | 98.8 | 99 |
| 8 | 98.9 | 98.9 | 99 |
| 9 | 98.8 | 98.9 | 98.8 |
| 10 | 98.8 | 98.8 | 98.8 |
| 11 | 98.9 | 98.9 | 99 |
| 12 | 98.9 | 98.7 | 98.8 |
| Control | 98.7 | 99.1 | 98.9 |

**TABLE 15**

| High-Molecular Weight Content (HMW %) | | | |
|---|---|---|---|
| Form. No. | Control | Agitation | Freeze-thaw |
| 1 | 0.7 | 0.9 | 0.7 |
| 2 | 0.8 | 0.7 | 0.6 |
| 3 | 0.9 | 0.8 | 0.8 |
| 4 | 0.7 | 0.8 | 0.7 |
| 5 | 0.7 | 0.7 | 0.7 |
| 6 | 0.8 | 0.8 | 0.8 |
| 7 | 0.9 | 1 | 0.9 |
| 8 | 0.9 | 0.9 | 0.8 |
| 9 | 1 | 1 | 1 |
| 10 | 1 | 0.9 | 1 |
| 11 | 0.9 | 0.8 | 0.8 |
| 12 | 1 | 1 | 1 |
| Control | 0.9 | 0.8 | 0.9 |

**TABLE 16**

| Low-Molecular Weight Content (LMW %) | | | |
|---|---|---|---|
| Form. No. | Control | Agitation | Freeze-thaw |
| 1 | 0.2 | 0.3 | 0.4 |
| 2 | 0.2 | 0.3 | 0.2 |
| 3 | 0.1 | 0.2 | 0.3 |
| 4 | 0.2 | 0.2 | 0.2 |
| 5 | 0.3 | 0.2 | 0.2 |
| 6 | 0.3 | 0.3 | 0.3 |
| 7 | 0.2 | 0.3 | 0.2 |
| 8 | 0.3 | 0.2 | 0.2 |
| 9 | 0.3 | 0.2 | 0.2 |
| 10 | 0.3 | 0.3 | 0.2 |
| 11 | 0.2 | 0.3 | 0.2 |
| 12 | 0.2 | 0.3 | 0.2 |
| Control | 0.3 | 0.1 | 0.3 |

As shown in Tables 14 to 16, the formulations using succinate buffer (1-6) generally showed higher stability than those using acetate buffer (7-12) under all tested conditions. Additionally, stability was found to be similar when using 0.01% or 0.1% PS20. Also, under normal conditions (control), formulations containing mannitol alone (3, 6, 9, 12) showed slightly lower stability compared to those additionally containing glycine or glycerol (1, 2, 4, 5, 7, 8, 10, 11).

### EXAMPLE 7. Evaluation for Long-term Freezing Stability of Formulations Containing Mannitol, Glycerol, and Glycine

### 7.1. Preparation of Formulations

Based on the results of Example 5, 8 formulations containing various combinations of mannitol, glycerol, and glycine as excipients were prepared.

In brief, the buffer was set to be 10 mM succinate, and the surfactant was either 0.01% (w/v) PS20 or 0.01% (w/v) PS80. The excipient was selected from mannitol, glycine, and glycerol, and the pH was fixed at 5.2, leading to the preparation of a total of 8 liquid formulations. As a control, a formulation identical to Humira (Humira; AbbVie) with 42 mg/ml mannitol, 0.1% PS80, pH 5.2 was prepared for comparative evaluation.

The formulations were prepared according to the composition shown in Table 17 below, using the in-house constructed adalimumab (concentration of adalimumab in the formulations: 100 mg/ml).

The conditions of the 8 prepared formulations are summarized in Table 17 below:

**TABLE 17**

| Form. No. | Buffer | pH | Excipient | Surfactant |
|---|---|---|---|---|
| 1 | 10 mM succinate | 5.2 | 11mM(0.2%) Mannitol, 266mM(2%) Glycine | 0.01% PS20 |
| 2 | 10 mM succinate | 5.2 | 55mM(1%) Mannitol, 222mM(1.67%) Glycine | 0.01% PS20 |
| 3 | 10 mM succinate | 5.2 | 277mM(2.08%) Glycine | 0.01% PS20 |
| 4 | 10 mM succinate | 5.2 | 11mM(0.2%) Mannitol, 239mM(2.2%) Glycerol | 0.01% PS20 |
| 5 | 10 mM succinate | 5.2 | 55mM(1%) Mannitol, 195mM(1.8%) Glycerol | 0.01% PS20 |
| 6 | 10 mM succinate | 5.2 | 250mM(2.3%) Glycerol | 0.01% PS20 |
| 7 | 10 mM succinate | 5.2 | 11mM(0.2%) Mannitol, 266mM(2%) Glycine | 0.01% PS80 |
| 8 | 10 mM succinate | 5.2 | 11mM(0.2%) Mannitol, 239mM(2.2%) Glycerol | 0.01% PS80 |
| Control | No buffer | 5.2 | 42 mg/mL mannitol | 0.1% PS80 |

| | | | | |
|---|---|---|---|---|
| ("%": %(w/v)) | | | | |

### 7.2. Long-term Freezing Stability Evaluation

The 8 formulations and the control formulation prepared in Example 7.1 were evaluated for long-term freezing stability by examining the pure charge difference characteristics (charge variants) on the surface of antibodies in the formulations using cation exchange high-performance liquid chromatography (CEX-HPLC). This evaluation of charge variants was performed using cation exchange high-performance liquid chromatography (CEX-HPLC) with reference to the method in in Reference Example 2.4, to measure changes in the main peak content (%), acidic variant content (Acidic Variants, Pre-Peaks; %), and basic variant content (Basic Variants, Post-Peaks; %).

After storing the 8 formulations and the control group formulation at -70°C for 3 months, adalimumab was diluted to a concentration of 1 mg/mL and subjected to CEX-HPLC to check for charge variants. The results obtained are presented in Table 18:

**TABLE 18**

| Form. No. | T=0 | | | T=3month | | |
|---|---|---|---|---|---|---|
| | Pre-Peaks | Main Peak | Post-Peaks | Pre-Peaks | Main Peak | Post-Peaks |
| 1 | 18.1 | 65.6 | 16.3 | 17.8 | 64.9 | 17.2 |
| 2 | 18 | 65.5 | 16.4 | 18.6 | 64.2 | 17.2 |
| 3 | 18.1 | 65.5 | 16.4 | 18.6 | 64.2 | 17.2 |
| 4 | 17.7 | 66 | 16.3 | 17.9 | 64.9 | 17.2 |
| 5 | 18 | 65.6 | 16.4 | 18.2 | 64.6 | 17.3 |
| 6 | 17.8 | 65.7 | 16.5 | 18.5 | 64.5 | 17.1 |
| 7 | 18.1 | 65.6 | 16.4 | 18.8 | 64 | 17.2 |
| 8 | 17.6 | 65.8 | 16.4 | 18.8 | 64 | 17.2 |
| Control | 17.9 | 65.7 | 16.4 | 18.3 | 64.7 | 17.1 |

As seen in the long-term stability results, formulations 1, 2, 4, and 5, particularly 1 and 4, exhibited relatively superior stability.

Additionally, after storing the 8 formulations and the control group formulation at -70°C for 3 months, Micro-Flow Imaging (MFI) (see Reference Example 2.3) was performed, and the results (Particles/mL) are presented in Table 19:

**TABLE 19**

| Form. No. | 1 | 2 | 3 | 4 | 5 | 6 | 7 | 8 | Control |
|---|---|---|---|---|---|---|---|---|---|
| >5 µm | 128 | 106 | 114 | 106 | 1382 | 473 | 608 | 123 | 117 |
| >10 µm | 27 | 23 | 18 | 17 | 334 | 101 | 43 | 25 | 35 |
| >25 µm | 0 | 0 | 0 | 0 | 21 | 8 | 0 | 8 | 4 |

It was observed that the formulations with added PS20 (formulations 1 to 4, especially 1 and 4) showed better results than those with added PS80 (formulations 7 and 8).

## Claims

1. A pharmaceutical composition, comprising:
80 mg/ml to 200 mg/ml of an anti-TNFα antibody;
a buffer;
an excipient; and
a surfactant, and
having a pH of 4 to 7.5,
wherein,
the buffer comprises at least one selected from the group consisting of succinate, phosphate, acetate, and pharmaceutically acceptable salts thereof,
the excipient comprises at least one selected from the group consisting of mannitol, glycerol, glycine, and pharmaceutically acceptable salts thereof, and
the surfactant comprises polysorbate 20.

2. The pharmaceutical composition of claim 1, wherein the buffer comprises at least one selected from the group consisting of succinate, phosphate, and pharmaceutically acceptable salts thereof.

3. The pharmaceutical composition of claim 2, wherein the buffer comprises succinate at a concentration of 10 mM or 20 mM, based on the total amount of the composition.

4. The pharmaceutical composition of claim 1, wherein the excipient comprises mannitol, a combination of mannitol and glycerol, or a combination of mannitol and glycine.

5. The pharmaceutical composition of claim 4, wherein the excipient comprises:
8.25mM to 14mM of mannitol and 239mM to 293mM of glycine; or
8.25mM to 14mM of mannitol and 217mM to 272mM of glycerol,
based on the total amount of the composition.

6. The pharmaceutical composition of claim 1, wherein the surfactant comprises polysorbate 20 at a concentration of 0.01 to 0.1% (w/v), based on the total amount of the composition.

7. The pharmaceutical composition of any one of claims 1 to 6, wherein, wherein the anti-TNFα antibody is adalimumab.

8. A method for producing a stable liquid formulation of an anti-TNFα antibody, the method comprises a step of mixing
80 mg/ml to 200 mg/ml of the anti-TNFα antibody;
a buffer;
an excipient; and
a surfactant to prepare an aqueous liquid composition,
wherein,
the buffer comprises at least one selected from the group consisting of succinate, phosphate, acetate, and pharmaceutically acceptable salts thereof,
the excipient comprises at least one selected from the group consisting of mannitol, glycerol, glycine, and pharmaceutically acceptable salts thereof,
the surfactant comprises polysorbate 20, and
the aqueous liquid composition has a pH of 4 to 7.5.

9. The method of claim 8, wherein the buffer comprises at least one selected from the group consisting of succinate, phosphate, and pharmaceutically acceptable salts thereof.

10. The method of claim 9, wherein the buffer comprises succinate at a concentration of 10 mM or 20 mM, based on the total amount of the composition.

11. The method of claim 8, wherein the excipient comprises mannitol, a combination of mannitol and glycerol, or a combination of mannitol and glycine.

12. The method of claim 11, wherein the excipient comprises:
8.25mM to 14mM of mannitol and 239mM to 293mM of glycine; or
8.25mM to 14mM of mannitol and 217mM to 272mM of glycerol, based on the total amount of the composition.

13. The method of claim 8, wherein the surfactant comprises polysorbate 20 at a concentration of 0.01 to 0.1 % (w/v), based on the total amount of the composition.

14. The method of any one of claims 8 to 13, wherein the anti-TNFα antibody is adalimumab.

15. A method for enhancing stability of an anti-TNFα antibody in a liquid formulation, the method comprising a step of mixing:
80 mg/ml to 200 mg/ml of the anti-TNFα antibody;
a buffer;
an excipient; and
a surfactant to prepare an aqueous liquid composition,
wherein,
the buffer comprises at least one selected from the group consisting of succinate, phosphate, acetate, and pharmaceutically acceptable salts thereof,
the excipient comprises at least one selected from the group consisting of mannitol, glycerol, glycine, and pharmaceutically acceptable salts thereof,
the surfactant comprises polysorbate 20, and
the aqueous liquid composition has a pH of 4 to 7.5.
